# EUROPÄISCHE PATENTSCHRIFT

(11) **EP 1 814 823 B1**
(45) Veröffentlichungstag und Bekanntmachung des Hinweises auf die Patenterteilung: **30.09.2009**
(21) Anmeldenummer: 05774216.5
(22) Anmeldetag: 18.08.2005
(51) Int. Cl.: C01C 1/04, C07C 273/10

(54) **VERFAHREN ZUR HERSTELLUNG VON HARNSTOFF AUS ERDGAS**
METHOD FOR THE PRODUCTION OF UREA FROM NATURAL GAS
PROCEDE DE PRODUCTION D'UREE A PARTIR DE GAZ NATUREL

(30) Priorität: 12.10.2004 DE 102004049774
(43) Veröffentlichungstag der Anmeldung: 08.08.2007
(73) Patentinhaber: Lurgi GmbH, 60439 Frankfurt am Main (DE)
(72) Erfinder: DAVEY, William, 60439 Frankfurt am Main (DE); WURZEL, Thomas, 61440 Oberursel/Ts (DE)
(74) Vertreter: Meyer-Dulheuer, Karl-Hermann
(86) Internationale Anmeldenummer: PCT/EP2005/008936
(87) Internationale Veröffentlichungsnummer: WO 2006/039960

(56) Entgegenhaltungen:
- EP-A- 0 905 127
- WO-A-02/38499
- GB-A- 2 048 840

## Beschreibung

Gegenstand der Erfindung ist ein zweistufiges Verfahren zur Herstellung von Harnstoff aus Erdgas, wobei in einer ersten Verfahrensstufe Ammoniak und Kohlendioxid erzeugt werden, die in einer zweiten Verfahrensstufe miteinander zu Harnstoff umgesetzt werden. Insbesondere betrifft die Erfindung die Herstellung von stöchiometrischen und überstöchiometrischen Mengen von Harnstoff aus Erdgas.

Es ist bekannt, dass die Herstellung von Harnstoff aus Erdgas in einem zweistufigen Verfahren durchgeführt werden kann.

In einer ersten Stufe wird Stickstoff als Ammoniak gebunden und gleichzeitig aus dem Erdgas Kohlendioxid erzeugt, während in einer zweiten Verfahrensstufe der Ammoniak mit dem Kohlendioxid zu Harnstoff umgesetzt wird. Es ist außerdem bekannt, dass die nach diesem Verfahren herstellbare Menge von Harnstoff durch die Menge des Kohlendioxids begrenzt ist, das in der ersten Verfahrensstufe gewonnen werden kann.

Üblicherweise wird in der ersten Verfahrensstufe, also der Ammoniaksynthese, aus Erdgas und einem sauerstoffhaltigen Gas, z.B. Luft, in einem Dampferzeuger ("steam reformer") ein Synthesegas erzeugt, und anschließend in einem weiteren Verfahrensschritt ("secondary reformer"), die Oxidation der im Erdgas enthaltenen Kohlenwasserstoffe weitgehend abgeschlossen. Dieses Verfahren führt zu einem Synthesegas, dessen Kohlendioxidgehalt etwas 10% geringer ist als zur Umsetzung des gleichzeitig entstandenen Ammoniaks zu Harnstoff stöchiometrisch erforderlich ist. Deshalb entsteht bei allen derartigen Verfahren zur Herstellung von Harnstoff das Problem, wie der überschüssige Ammoniak verwendet werden soll.

Es wurde nun ein neues Verfahren gefunden, bei dem durch eine partielle Oxidation des Erdgases die Menge des erzeugten Kohlendioxids gleich oder größer als die stöchiometrische Menge ist, die zur Umsetzung des Ammoniaks zur Harnstoff erforderlich ist. Mit diesem Verfahren ist es deshalb möglich, nicht nur den gesamten entstandenen Ammoniak zu Harnstoff umzusetzen, sondern es können sogar so große Mengen Kohlenstoffdioxid erzeugt werden, dass sie zur Umsetzung von zusätzlich aus einer externen Ammoniakquelle dazu gegebenen Ammoniak ausreichen, wodurch erfindungsgemäß eine erheblich höhere Menge Harnstoff als bei konventionellen Verfahren erzeugt werden kann.

Gegenstand der Erfindung ist deshalb ein Verfahren zu Herstellung von Harnstoff aus Erdgas, bei dem man
a) in einer ersten Verfahrensstufe Erdgas mit einem sauerstoffhaltigen Gas einer partiellen Oxidation oder eines authothermalen Reformieren unterwirft und das entstehende, im wesentlichen aus Kohlenmonoxid, Kohlendioxid, Methan und Wasserstoff bestehende rohe Synthesegas durch eine katalytische Konvertierung von CO und H₂O zu CO₂ und H₂ umwandelt, dann in einer mehrstufigen Gasreinigung Kohlendioxid, Kohlenmonoxid und Methan aus dem Synthesegas entfernt und den zurückbleibenden Wasserstoff nach Zusatz von Stickstoff katalytisch zu Ammoniak umsetzt, und anschließend
b) in einer zweiten Verfahrensstufe den Ammoniak mit dem zuvor abgetrennten Kohlendioxid wieder vereint und dabei den Ammoniak vollständig umsetzt.

Insbesondere wird erfindungsgemäß, in der zweiten Verfahrensstufe zusätzlich aus einer externen Ammoniakquelle soviel NH₃ dazuzugeben, dass das in der ersten Stufe gebildete, überschüssige Kohlendioxid vollständig zu Harnstoff umgesetzt werden kann.

Das in der ersten Verfahrensstufe erfindungsgemäß eingesetzte Verfahren zur katalytischen Erzeugung von Ammoniak aus einem Stickstoff Wasserstoff Gemisch ist in der deutschen Offenlegungsschrift 100 55 818 beschrieben worden. Dabei wird Erdgas zusammen mit einem sauerstoffreichen Gas in einen autothermen Reformer geleitet, wo man bei Temperaturen im Bereich von 900 bis 1200°C, einem Druck von 40 bis 100 bar und in Gegenwart eines Spaltkatalysators ein rohes Synthesegas erzeugt. Dieses Synthesegas weist, trocken gerechnet, einen H₂Gehalt von 55 bis 75 Volumenprozent, einen CO-Gehalt von 15 bis 30 Volumenprozent, einen CO₂Gehalt von 5 bis 30 Volumenprozent und ein Volumenverhältnis H₂:CO von 1,6 bis 4 auf. Das rohe Synthesegas wird dann aus dem autothermen Reformer abgezogen, gekühlt, durch eine katalytische Konvertierung zum Umwandeln von CO in H₂ geleitet und ein konvertiertes Synthesegas mit einem H₂-Gehalt, trocken gerechnet von mind. 55 Volumenprozent und einem CO-Gehalt von höchstens 8 Volumenprozent abgezogen. Das konvertierte Synthesegas wird dann einer mehrstufigen Gasreinigung zum Entfernen von CO₂, CO und CH₄ unterzogen und dabei ein Stickstoff-Wasserstoffgemisch erzeugt, weiches man katalytisch zu Ammoniak umsetzt.

Im Allgemeinen besteht das als Ausgangsmaterial eingesetzte Erdgas im Wesentlichen aus Methan. In diesem Fall wird die daraus gewonnene Menge an Kohlendioxid stöchiometrisch ausreichen, um den in der ersten Stufe des erfindungsgemäßen Verfahrens gebildeten Ammoniak in Harnstoff umzuwandeln. In diesem Fall wird also kein Überschuss an Ammoniak vorliegen. Falls jedoch das Ausgangsmaterial auch größere Mengen an höheren Kohlenwasserstoffen enthält, was häufig der Fall ist, dann übersteigt die Menge des in der ersten Stufe des erfindungsgemäßen Verfahrens gebildeten Kohlendioxids die stöchiometrisch erforderliche Menge um bis zu 10%. Das bedeutet, dass zusätzlicher Ammoniak der Reaktion zugeführt werden kann, entweder von einer benachbarten Anlage oder aus zugekauften Vorräten, wodurch dann die Menge des erfindungsgemäß gebildeten Harnstoff diejenige einer üblichen Anlage zur Ammoniakherstellung um bis zu 20% übersteigt. Hieraus erkennt man die erheblich verbesserte Wirtschaftlichkeit des erfindungsgemäßen Verfahrens zur Herstellung von Harnstoff gegenüber allen bisher bekannten Verfahren zur Herstellung von Harnstoff aus Erdgas.

Für die erste Stufe des erfindungsgemäßen Verfahrens ist es wichtig, dass man beim Erzeugen des rohen Synthesegases auf eine Anlage zum Dampfreformieren (steam reforming) verzichtet. Stattdessen wird erfindungsgemäß ein autothermer Reformer eingesetzt, der bei relativ hohen Drucken arbeitet, die im Bereich von 30 bis 100 bar, meistens zwischen 40 bis 80 bar liegen. Stromab vom autothermen Reformer kann dieser hohe Druck ungefähr beibehalten werden, so dass man das Synthesegas vor Eintritt in die Ammoniaksynthese nur wenig verdichten muss. Dies ist gegenüber konventionellen Arbeitsweisen mit Dampfreformierung, in welcher nur relativ niedrige Drucke zugelassen sind, erheblich kostengünstiger. Der autotherme Reformer hat gegenüber der Dampfreformierung den weiteren Vorteil, dass er ein Gas mit einem ausreichenden H₂/CO₂-Verhältnis liefert, so dass man - wie bereits oben erwähnt - nach der Konvertierung mit dem in der Gasreinigung anfallenden CO₂ das gesamte hergestellte NH₃ zur Harnstoff umwandeln kann.

Bei dem erfindungsgemäßen Verfahren wird also in der ersten Verfahrensstufe eine katalytische Konvertierung durchgeführt, bei der Kohlenmonoxid und H₂O zu CO₂ und H₂ umgewandelt werden. Daran schließt sich dann eine mehrstufige Gasreinigung zur Entfernung von Kohlendioxid, Kohlenmonoxid und Methan an. Besonders vorteilhaft ist ein Waschverfahren, bei dem man z.B. Methanol bei Temperaturen von -20 bis -70°C einsetzt. Hierbei wird nur relativ wenig Energie, einschließlich Kompressionsenergie, verbraucht.

Es ist zweckmäßig, wenn das dem autothermen Reformer zugeführte sauerstoffreiche Gas einen Sauerstoffgehalt von mindestens 70 Volumenprozent und im allgemeinen sogar mindestens 90 Volumenprozent aufweist. Auf diese Weise verringert man den Gehalt an Verunreinigung im rohen Synthesegas und die Waschstufe kann verkleinert werden. Das die Konvertierung verlassende Synthesegas hat vorzugsweise ein Volumenverhältnis von Wasserstoff zu CO₂ von 2 bis 3 (trocken gerechnet).

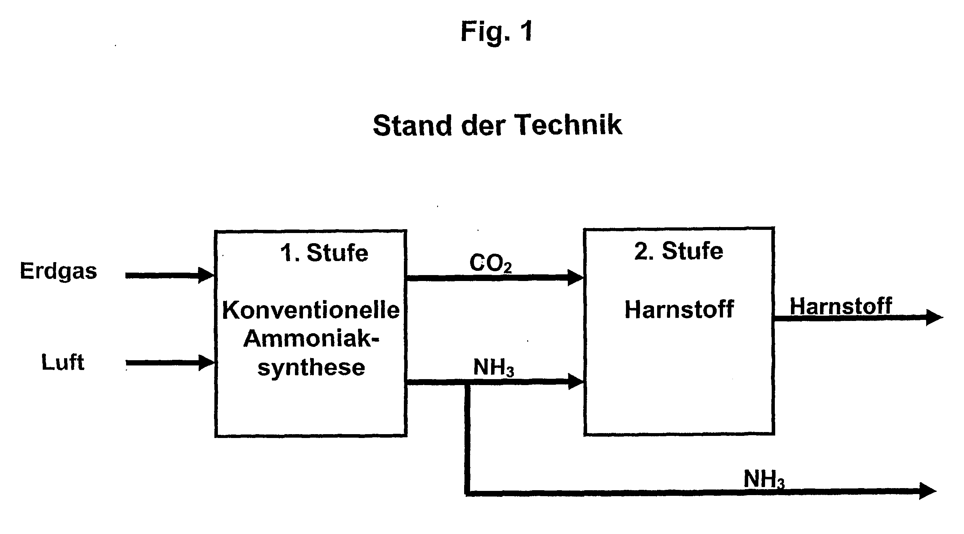

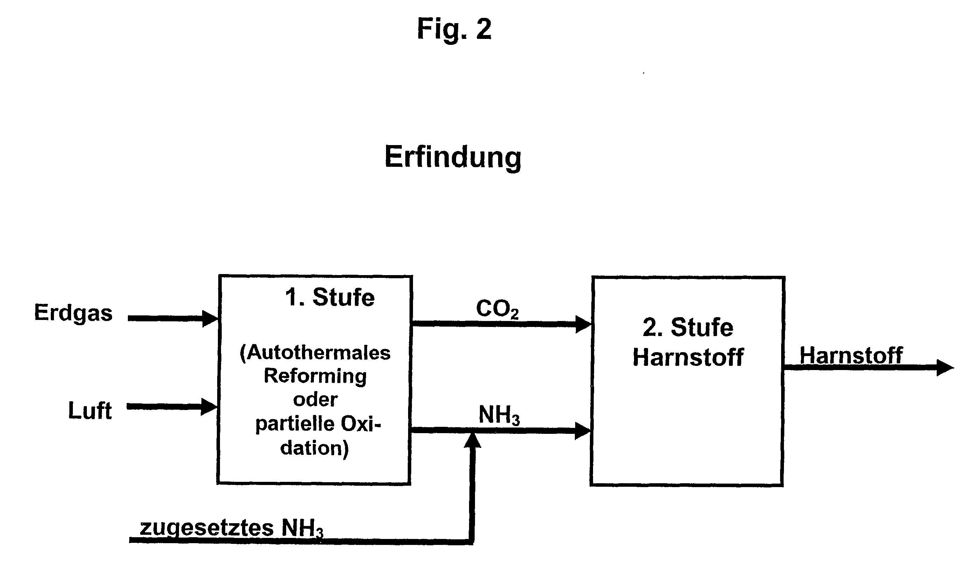

Die als Fig.1 und Fig. 2 beigefügten Blockschemata zeigen die Vorteile des erfindungsgemäßen Verfahrens gegenüber einem Verfahren nach dem Stand der Technik. Im Fig. 1 ist der Stand der Technik dargestellt. Nach diesem Verfahren wird aus Erdgas und Luft in der ersten Verfahrensstufe ein Gasgemisch aus Ammoniak und Kohlendioxid hergestellt, das allerdings 10% weniger Kohlendioxid enthält, als zur stöchiometrischen Umsetzung zu Harnstoff erforderlich ist. Überschüssiges Ammoniak muss deshalb aus dem System entfernt werden. Kennzeichnend für das konventionelle Verfahren sind das Dampfreformieren und eine nachfolgende zweite Reformierung.

Fig. 2 zeigt dagegen das erfindungsgemäße Verfahren, bei dem eine partielle Oxidation oder ein autothermales Reformieren eingesetzt wird, wie es in der deutschen Patentanmeldung 100 55 818 beschrieben ist. Bei diesem Verfahren kann von außen zugesetztes Ammoniak eingesetzt werden, um das im Überschuss gebildete Kohlendioxid zu binden. Hierdurch werden erhebliche Mengen von zusätzlichem Harnstoff gebildet.

Besondere Vorteile des erfindungsgemäßen Verfahrens ergeben sich dann, wenn das erfindungsgemäße Verfahren z. B. mit einer benachbarten Anlage zur Methanolsynthese aus Erdgas kombiniert wird. In einer derartigen Anlage wird reichlich überschüssiges Kohlendioxid gebildet, dass dem erfindungsgemäßen Verfahren zusätzlich zugeführt und mit weiterem aus einer externen Quelle zugesetztem Ammoniak zu einer weiteren Erhöhung der Ausbeute an Harnstoff beiträgt.

## Patentansprüche

1. Verfahren zur Herstellung von Harnstoff aus Erdgas, **dadurch gekennzeichnet, dass** man
a) in einer ersten Verfahrensstufe Erdgas mit einem sauerstoffhaltigen Gas einer partiellen Oxidation oder eines autothermalen Reformieren unterwirft und das entstehende, im wesentlichen aus Kohlenmonoxid, Kohlendioxid, Methan und Wasserstoff bestehende rohe Synthesegas dann durch eine katalytische Konvertierung von CO und H₂O zu CO₂ und H₂ umwandelt, dann in einer mehrstufigen Gasreinigung Kohlendioxid, Kohlenmonoxid und Methan entfernt und den Wasserstoff nach Zusatz von Stickstoff katalytisch zu Ammoniak umsetzt, und anschließend
b) in einer zweiten Verfahrensstufe den Ammoniak mit dem zuvor abgetrennten Kohlendioxid wieder vereint und zusätzlich aus einer externen Ammoniakquelle soviel NH₃ dazugibt, dass das Kohlendioxid vollständig in Harnstoff umgesetzt werden kann.

2. Verfahren nach Anspruch 1, **dadurch gekennzeichnet, dass** das für die partielle Oxydation eingesetzte sauerstoffhaltige Gas einen Sauerstoffgehalt von mindestens 70 Volumenprozent aufweist.

3. Verfahren nach den Ansprüchen 1 und 2, **dadurch gekennzeichnet, dass** das die Konvertierung verlassende Synthesegas ein Volumenverhältnis von Wasserstoff : CO₂ von 2 bis 3 (trocken gerechnet) aufweist.

## Claims

1. A method for the production of urea from natural gas, **characterized in that**
a) in a first method step natural gas undergoes a partial oxidation or an autothermal reformation with a gas containing oxygen, and the resulting raw synthesis gas, consisting substantially of carbon monoxide, carbon dioxide, methane and hydrogen, is then transformed by a catalytic conversion of CO and H₂O to CO₂ and H₂, then carbon dioxide, carbon monoxide and methane are removed in a multistep gas cleaning process and the hydrogen is converted to ammonia after the addition of nitrogen, and then
b) in a second method step, the ammonia is recombined with the previously separated carbon dioxide and in addition from an external ammonia source as much NH₃ is added such that the carbon dioxide can be fully converted into urea.

2. The method according to Claim 1, **characterized in that** the oxygen-containing gas which is used for the partial oxidation has an oxygen content of at least 70 percent by volume.

3. The method according to Claims 1 and 2, **characterized in that** the synthesis gas leaving the conversion has a volume ratio of hydrogen : CO₂ of 2 to 3 (calculated dry).

## Revendications

1. Procédé de préparation d'urée à partir de gaz naturel, **caractérisé en ce que**
a) dans une première étape, on soumet du gaz naturel à une oxydation partielle par un gaz contenant de l'oxygène ou à un reformage autothermique et qu'on transforme le gaz de synthèse brut obtenu, constitué essentiellement de monoxyde de carbone, de dioxyde de carbone, de méthane et d'hydrogène, par convertissement catalytique de CO et de H₂O en CO₂ et H₂ pour ensuite éliminer, dans un dispositif de purification de gaz à plusieurs étages, le dioxyde de carbone, le monoxyde de carbone et le méthane et transformer l'hydrogène en ammoniaque par réaction catalytique après ajout d'azote, et puis
b) dans une deuxième étape, on réunit ledit ammoniaque avec le dioxyde de carbone précédemment séparé et qu'on ajoute suffisamment de NH₃ issu d'une source d'ammoniaque extérieure pour permettre de complètement transformer le dioxyde de carbone en urée.

2. Procédé selon la revendication 1, **caractérisé en ce que** le gaz contenant de l'oxygène, qui est mis en oeuvre dans ladite oxydation partielle, présente une teneur en oxygène d'au moins 70 % en volume.

3. Procédé selon les revendications 1 et 2, **caractérisé en ce qu'**à l'issue du processus de convertissement, le gaz de synthèse présente un rapport volumique entre hydrogène : CO₂ compris entre 2 et 3 (calculé à l'état sec).
